# EUROPEAN PATENT APPLICATION

(11) **EP 2 856 868 A1**
(43) Date of publication of application: **08.04.2015**
(21) Application number: 13797068.7
(22) Date of filing: 30.05.2013
(51) Int. Cl.: A01K 67/00, A23K 1/00, A23K 1/18

(54) **METHOD FOR DETERMINING AUXOTROPHY OF FISH**

(30) Priority: 30.05.2012 JP 2012123399
(71) Applicant: Ajinomoto Co., Inc., Tokyo, 104-8315 (JP)
(72) Inventor: TAKIMOTO, Tetsuya, Kawasaki-shi Kanagawa 210-8681 (JP)
(74) Representative: Hoffmann Eitle
(86) International application number: PCT/JP2013/065066
(87) International publication number: WO 2013/180226

(57) **Abstract**

A means for determining the nutritional requirements of fish is provided. The nutritional requirements of fish is determined by comparing the amino acid sequences of proteins predicted from genome information of fish with the amino acid sequences of known proteins thereby to estimate the functions of the respective proteins of the fish, identifying a metabolic pathway present in the fish on the basis of the estimated functions of the respective proteins, and determining the nutritional requirements of the fish on the basis of a difference between the identified metabolic pathway present in the fish and a metabolic pathway present in a reference organism.

## Description

### Technical Field

The present invention relates to a method for determining the nutritional requirements of fish. This method is useful in the fields of cultivation of fish, and so forth.

### Background Art

Fish is a highly nutritious food containing good-quality proteins and lipids. Moreover, fish is also useful as a research material.

In recent years, in view of preservation of the marine ecosystem, the stable supply of marine resources, etc., a conversion of "catch fishery" to "culture fishery" has been attempted and the cultivation of fish is actively practiced.

For the cultivation of fish, the development of feeds showing a high growth promoting property is strongly desired. Mixed feeds that are currently widely used have been developed on the basis of feeds naturally taken by fish (small fish etc.) as a basic composition. The mixed feeds are produced by, for example, granulating raw materials such as fish meal, fats and oils, cereals, chaff and bran, vitamins, and minerals using a pellet machine or an extruder.

Since it is considered that the nutritional requirements of fish differ depending on the species of fish, it is desirable to develop mixed feeds so that they meet the nutritional requirements of fish to be cultivated. For humans, for example, there are papers discussing the possibility of predicting the nutritional requirements on the basis of genome information (Non-patent documents 1 and 2). However, there is no known method for predicting the nutritional requirements of fish.

### Prior Art References

### Non-patent documents

Non-patent document 1: Tamura T, et al., Genome Inform., 22:176-90, Jan. 2010
Non-patent document 2: Romero P, et al., Genome Biol., 2005;6(1):R2. Epub, Dec. 22, 2004.

### Summary of the Invention

### Object to be Achieved by the Invention

An object of the present invention is to provide a means for determining the nutritional requirements of fish.

### Means for Achieving the Object

The inventor of the present invention conducted various researches in order to achieve the aforementioned object. As a result, the inventor found that by annotating proteins of *Takifugu rubripes* (tiger pufferfish) and *Homo sapience* (human) with functions estimated on the basis of the amino acid sequences of the proteins predicted from the genome information, identifying metabolic pathways present in *Takifugu rubripes* and *Homo sapience* on the basis of the result of the annotation, and comparing them, candidates for nutrients specifically and strongly required by *Takifugu rubripes* could be determined, and accomplished the present invention.

Thus, the present invention is embodied, for example, as follows.
[1] A method for determining nutritional requirements of fish, comprising:
   (1) comparing the amino acid sequences of proteins predicted from genome information of fish with the amino acid sequences of known proteins thereby to estimate the functions of the respective proteins of the fish;
   (2) identifying a metabolic pathway present in the fish on the basis of the estimated functions of the respective proteins; and
   (3) determining nutritional requirements of the fish on the basis of a difference between the identified metabolic pathway present in the fish and a metabolic pathway present in a reference organism,
   wherein the reference organism is a mammal or a bird.
[2] The method as mentioned above, which comprises: comparing the amino acid sequences of proteins predicted from genome information of the reference organism with the amino acid sequences of known proteins thereby to estimate the functions of the respective proteins of the reference organism; and identifying the metabolic pathway present in the reference organism on the basis of the estimated functions of the respective proteins.
[3] The method as mentioned above, wherein when a certain metabolic pathway is not present in the fish, but it is present in the reference organism, it is determined that the fish strongly requires a metabolite generated via the metabolic pathway.
[4] The method as mentioned above, wherein the amino acid sequences are described in the FASTA format.
[5] The method as mentioned above, wherein the functions of the respective proteins are estimated by homology search with known proteins using BLAST.
[6] The method as mentioned above, wherein the functions of the respective proteins are estimated by annotation based on KEGG Orthology (KO).
[7] The method as mentioned above, wherein the estimated functions of the respective proteins are mapped on a metabolic pathway.
[8] The method as mentioned above, wherein the reference organism is *Homo sapience.*
[9] A method for producing feed for fish, which comprises: determining metabolite(s) strongly required by the fish according to the aforementioned method; and adding the metabolite(s) to a raw material of feed.

### Modes for Carrying out the Invention

Hereafter, the present invention will be explained in detail.

### <1> Method of the present invention

The method of the present invention is a method for determining the nutritional requirements of fish.

The method of the present invention comprises (1) comparing the amino acid sequences of proteins predicted from genome information of fish with the amino acid sequences of known proteins thereby to estimate the functions of the respective proteins of the fish, (2) identifying a metabolic pathway present in the fish on the basis of the estimated functions of the respective proteins, and (3) determining the nutritional requirements of the fish on the basis of a difference between the identified metabolic pathway present in the fish and a metabolic pathway present in a reference organism.

In the present invention, the "fish" is not particularly limited, so long as it is fish of which the genome sequence has been determined, and of which the amino acid sequences of proteins predicted from the genome information are available. The expression that "the genome sequence has been determined, and the amino acid sequences of proteins predicted from the genome information are available" include a case where the amino acid sequences of proteins predicted from genome information are published, as well as a case where the amino acid sequences of proteins can be obtained by genome analysis performed personally or performed by outsourcing or the like. As of May 29, 2013, specific examples of fish for which the amino acid sequences of proteins deduced from genome information are available include *Takifugu rubripes* (tiger pufferfish), *Tetraodon nigroviridis* (green spotted pufferfish), *Danio rerio* (zebrafish), *Gasterosteus aculeatus* (three-spined stickleback, bristling), *Oryzias latipes* (cyprinodont), *Gadus morhua* (Atlantic cod), *Petromyzon marinus* (lamprey), *Latimeria chalumnae* (coelacanth), *Xiphophorus maculatus* (platyfish), and *Oreochromis niloticus* (Nile tilapia).

In the present invention, the "reference organism" is a mammal or a bird. The "reference organism" is not particularly limited, so long as it is a mammal or a bird of which a metabolic pathway has been identified or can be identified. Examples of the mammal or bird of which a metabolic pathway can be identified include mammals and birds of which the genome sequence is determined, and of which the amino acid sequences of proteins predicted from the genome information is available. A metabolic pathway present in the reference organism can be appropriately identified on the basis of the amino acid sequences of proteins predicted from the genome information. That is, the method of the present invention can comprise comparing the amino acid sequences of proteins predicted from genome information of a reference organism with the amino acid sequences of known proteins thereby to estimate the functions of the respective proteins of the reference organism, and can comprise identifying a metabolic pathway present in the reference organism on the basis of the estimated functions of the respective proteins. The reference organism is preferably an organism of which the nutritional requirements are well known. Specific examples of the mammal or bird for which the amino acid sequences of proteins predicted from genome information are available, and of which the nutritional requirements are well known include, for example, human, mouse, and fowl. Human is especially preferred.

The amino acid sequences of a proteins can be obtained from, for example, a publicly known database. Although such a database is not particularly limited, examples thereof include, for example, NCBI (http://www.ncbi.nlm.nih.gov/), Ensembl (http://asia.ensembl.org/index.html), DDBJ (DNA Data Bank of Japan, http://www.ddbj.nig.ac.jp/), and EMBL (European Molecular Biology Laboratory, http://www.embl.org/). Specifically, for example, on the ftp site of Ensembl (http://asia.ensembl.org/info/data/ftp/index.html), by selecting "Protein sequence (FASTA)" of an objective biological species, the amino acid sequences of all the proteins of that biological species predicted from the genome information can be downloaded in the FASTA format. Amino acid sequence(s) used in the method of the present invention can also be referred to as "amino acid sequence data". The method of the present invention can comprise a step of downloading and obtaining such amino acid sequence data.

Amino acid sequence data of a protein can consist of amino acid sequence data deduced from genome information (nucleotide sequence), or can contain other amino acid sequence data. For example, amino acid sequence data of a protein can contain experimentally determined amino acid sequence data.

It is sufficient that amino acid sequence data of a protein is described in a format usable for estimating the function of the protein. The "format usable for estimating the function of a protein" can be appropriately chosen depending on software or an algorithm thereof used for estimating the function of the protein. For example, the amino acid sequence of a protein is preferably described in the FASTA format. The FASTA format is a format containing one or more pieces of sequence data (for example, amino acid sequence data of protein(s)) divided by header line(s). FASTA format including two or more pieces of sequence data is also called multi-FASTA format. The multi-FASTA format is especially useful for annotating two or more kinds of proteins at once. Specifically, for example, when the annotation is performed with KAAS described later, amino acid sequences described in the multi-FASTA format can be batch-processed.

The function of a protein can be estimated by comparing the amino acid sequence of the protein with the amino acid sequence(s) of known protein(s). The "known protein" is not particularly limited so long as it is a protein or a functional motif of which the amino acid sequence and function are known. The amino acid sequence of a known protein can be an experimentally determined amino acid sequence, or can be an amino acid sequence estimated from genome information or from a gene sequence. The function of a known protein can be an experimentally determined function, or can be a function estimated by comparison with the amino acid sequence(s) of other known protein(s). The function of a protein can be estimated by, for example, comparing the amino acid sequence of the protein with the amino acid sequence(s) of already annotated protein(s) registered at a publicly known database. Comparison of amino acid sequences can be performed by, for example, homology search, motif prediction, or the like. Homology search can be performed by using, for example, BLAST.

Specifically, for example, amino acid sequence data of a protein described in the FASTA format can be processed on KAAS (KEGG automatic annotation server) of KEGG (Kyoto Encyclopedia of Genes and Genomes, http://www.genome.jp/kegg/) or of iKeg, which is a local server of KEGG, and annotated on the basis of KO (KEGG Orthology), so as to estimate the function of the protein. In such a case, annotation of an objective protein is performed by comparison with the amino acid sequence(s) of known protein(s) to which annotation based on KO on the KEGG database has been added. As for the details of KEGG, for example, Kanehisa M, et al., Nucleic Acids Res., 34, D354-357 (2006), or Kanehisa M, et al., Nucleic Acids Res., 36, D480-D484 (2008) can be referred to. As for the details of KAAS, for example, Moriya Y, et al., Nucleic Acids Res., 35 (Web Server issue):W182-5, Jul. 2007 can be referred to. Processing of amino acid sequence data can be performed for each protein individually, or for two or more kinds of proteins at once. For example, by processing data including the amino acid sequences of two or more kinds of proteins described in the multi-FASTA format on KAAS, annotation of the two or more kinds of proteins based on KO can be performed at once. In the method of the present invention, either one of the estimation of the function of a protein of fish and the estimation of the function of a protein of a reference organism can be performed first, or both of them can be performed simultaneously.

In addition, the method for estimating the function of a protein is not limited to the annotation based on KO, and the function of a protein can be estimated by any method that enables functional classification of a protein based on amino acid sequence. For example, the function of a protein can be estimated by comparison (for example, homology search) with the amino acid sequence(s) of already annotated protein(s) of Swiss-Prot or TrEMBL in UniProtKB (UniProt Knowledgebase, http://www.uniprot.org/help/uniprotkb).

In addition, "estimating the function of a protein by comparing the amino acid sequence of the protein with the amino acid sequence(s) of known protein(s)" includes estimation by direct processing of amino acid sequence data of the protein, as well as estimation by processing of arbitrary data that can be converted into the amino acid sequence of the protein. That is, in the present invention, the function of a protein can be estimated on the basis of arbitrary data that can be converted into the amino acid sequence of the protein. Examples of such data include, for example, the nucleotide sequence of a gene. Such data can be converted into the amino acid sequence of a protein beforehand or at the time of estimation of the function (at the time of annotation), and then can be used.

In the present invention, a "protein estimated to have a (certain) function" can also be called "protein annotated with a (certain) function". Proteins each of which the function was not estimated in the above estimation step may not be involved in the following steps (such as identification of a metabolic pathway).

In the method of the present invention, a metabolic pathway present in fish is identified on the basis of the functions of the respective proteins estimated as described above. That is, when objective fish has a protein annotated with the function corresponding to a certain metabolic pathway, it can be concluded that the fish has that metabolic pathway. Also, when objective fish does not have any protein annotated with the function corresponding to a certain metabolic pathway, it can be concluded that the fish does not have that metabolic pathway. Furthermore, a metabolic pathway present in a reference organism can also be identified in a similar manner. In the present invention, a "metabolic pathway" can consist of a single reaction step, can consist of two or more sequential reaction steps, or can consist of a combination thereof. The "function corresponding to a metabolic pathway" refers to one or more kinds of functions for catalyzing one or more reaction steps constituting the metabolic pathway. Also, the "protein annotated with the function corresponding to a metabolic pathway" refers to one or more kinds of proteins annotated with such one or more kinds of functions.

The method of the present invention can comprise the step of mapping the functions of the respective proteins estimated as described above on a metabolic pathway. Specifically, for example, at first, the function(s) of protein(s) common to fish and a reference organism, and the function(s) of protein(s) specific to either one of the fish and the reference organism are distinguished on the basis of the result of the aforementioned annotation with function. Such distinction can be performed manually or automatically. For example, such distinction can be automatically performed by using statistical analysis software. Specific examples of such statistical analysis software include, for example, the open source statistical analysis software "R" (http://www.r-project.org/). By mapping the distinguished functions of the proteins on a metabolic pathway, metabolic pathway(s) common to the fish and the reference organism, and metabolic pathway(s) specific to either one of them can be visualized. By such visualization, comparison of the metabolic pathways of the reference organism and fish becomes easier. Such mapping can be performed by using, for example, the KEGG mapper (http://www.genome.jp/kegg/mapper.html).

In the method of the present invention, the nutritional requirements of fish are determined on the basis of the difference between the above-identified metabolic pathway(s) present in the fish and metabolic pathway(s) present in a reference organism. For example, the identified metabolic pathways can be separated into metabolic pathway(s) common to fish and a reference organism, and metabolic pathway(s) specific to either one of the fish and the reference organism, and the nutritional requirements of the fish can be determined on the basis of the metabolic pathway(s) specific to either one of them. That is, when the presence or absence of a certain metabolic pathway differs between fish and a reference organism, it can be concluded that the nutritional requirement for a metabolite generated via that metabolic pathway differs between the fish and the reference organism. Specifically, for example, when a certain metabolic pathway is not present in fish, but it is present in a reference organism (i.e. when the metabolic pathway is specific to the reference organism), it can be determined that the fish strongly requires a metabolite generated via the metabolic pathway. The phrase "fish strongly requires a metabolite" means that the fish requires the metabolite strongly compared with the reference organism. The phrase "fish strongly requires a metabolite" can also mean, for example, that the fish requires a supply of the metabolite from outside while the reference organism does not require a supply of the metabolite from outside. Also, for example, when a certain metabolic pathway is not present in a reference organism, but it is present in fish (i.e. when the metabolic pathway is specific to the fish), it can be determined that the fish weakly requires a metabolite generated via the metabolic pathway. The phrase "fish weakly requires a metabolite" means that the fish requires the metabolite weakly compared with the reference organism. The phrase "fish weakly requires a metabolite" can also mean, for example, that the fish does not require a supply of the metabolite from outside while the reference organism requires a supply of the metabolite from outside.

A "metabolite generated via a metabolic pathway" includes a direct product of the metabolic pathway, as well as a metabolite generated from the product by further metabolism. The range of the "metabolite generated from the product by further metabolism" can vary depending on the kind of the metabolic pathway and the presence or absence of a bypass pathway. The range of the "metabolite generated from the product by further metabolism" can be a range to 20^{th}, 10^{th}, 5^{th}, or 3^{rd} compound downstream of the direct product of the metabolic pathway as 1^{st} compound.

In particular, for example, when a metabolic pathway that generates a metabolite important for growth or biological activities of a reference organism is deficient in fish, or when supply of a metabolite that serves as a substrate of a metabolic pathway specific to fish is insufficient, it can be concluded that the metabolite and/or a metabolite generated from the metabolite by further metabolism are/is necessary to be acquired from the outside, and the metabolite(s) can be selected as candidate(s) for nutrient(s) strongly required by the fish.

In the method of the present invention, identification and comparison of a metabolic pathway can be performed for all the proteins annotated with functions, or for a part of the proteins annotated with functions. Furthermore, in the method of the present invention, comparison between fish and a reference organism can be performed for all the identified metabolic pathways, or for a part of the identified metabolic pathways. For example, for determining the nutritional requirement for a specific metabolite in fish, it is sufficient that identification and comparison of a metabolic pathway are performed for protein(s) annotated with the function(s) relating to the specific metabolite. The "function relating to a specific metabolite" means the function corresponding to a metabolic pathway that generates the specific metabolite, or the function corresponding to a metabolic pathway that generates a metabolite that serves as a substrate of the metabolic pathway that generates the specific metabolite. Specific examples of the specific metabolite include, for example, saccharides, amino acids, lipids, and vitamins.

Whether the nutritional requirements of fish have been correctly determined by the method of the present invention can be confirmed by feeding the fish with each of feed produced according to the nutritional requirements (for example, feed containing metabolite(s) determined to be strongly required by the fish) and control feed (for example, feed not containing the metabolite(s)), and comparing the degrees of growth obtained with the respective feeds.

### <2> Program of the present inventions

The program of the present invention is a program for making a computer execute the respective steps of the method of the present invention.

That is, one embodiment of the program of the present invention is a program for making a computer execute the following steps (1) to (3):
(1) a step of comparing the amino acid sequences of proteins predicted from genome information of fish with the amino acid sequences of known proteins thereby to estimate the functions of the respective proteins of the fish,
(2) a step of identifying a metabolic pathway present in the fish on the basis of the estimated functions of the respective proteins, and
(3) a step of determining nutritional requirements of the fish on the basis of a difference between the identified metabolic pathway present in the fish and the metabolic pathway present in a reference organism.

Furthermore, the program of the present invention can make a computer execute a step of comparing the amino acid sequences of proteins predicted from genome information of the reference organism with the amino acid sequences of known proteins thereby to estimate the functions of the respective proteins of the reference organism, and can make a computer execute a step of identifying a metabolic pathway of the reference organism on the basis of the estimated functions of the respective proteins.

Also, for example, the program of the present invention can further make a computer execute a step of downloading and obtaining amino acid sequence data. Also, for example, the program of the present invention can further make a computer execute a step of mapping the result of annotation with function on a metabolic pathway.

The program of the present invention can be recorded on a recording medium readable by a computer, and provided. A "recording medium readable by a computer" recited herein refers to a recording medium on which information such as data and program can be stored by electric action, magnetic action, optical action, mechanical action, chemical action, or the like, and from which the stored information can be read by a computer. Examples of such a recording medium include, for example, floppy disc (registered trademark), magnetic optical disc, CD-ROM, CD-R/W, DVD-ROM, DVD-R/W, DVD-RAM, DAT, 8 mm tape, memory card, hard disk, ROM (read only memory), SSD, and so forth. As for the program of the present invention, the respective steps to be executed by a computer can be recorded as a single program collectively, or can be recorded as separate programs individually or as an arbitrary combination of separate programs.

### <3> Use of the method of the present inventions

Feed for fish can be produced by referring to the nutritional requirements of the fish determined by the method of the present invention. For example, feed can be produced by adding a metabolite determined to be strongly required by fish according to the method of the present invention to a raw material. Also, for example, feed can be produced with reducing the blending amount of a metabolite determined to be weakly required by fish according to the method of the present invention. Such feed can be produced by using the same raw materials and methods as those used for producing usual feed for fish, except that such a specific metabolite as mentioned above is added to a raw material and/or the blending amount of such a specific metabolite as mentioned above is reduced. Thus, feed containing such a specific metabolite as mentioned above and/or having a reduced blending amount of such a specific metabolite as mentioned above can be obtained. Feed produced as described above can be suitably used for cultivation (farming) of fish.

For example, a usually used raw material of feed for fish, such as liquid feed, powder feed, solid feed, moist pellet, dry pellet, extruded pellet, and live bait, can be appropriately chosen and prepared, and a metabolite determined to be strongly required by objective fish according to the method of the present invention can be blended. As raw materials for preparing such a raw material of feed, fish meal, bone meal, skim milk, cottonseed meal, wheat flour, wheat germ, rice bran, brewer's yeast, vitamins, soybean meal, plant residue, and so forth are generally used.

### Example

The present invention will be further specifically explained with reference to the following example. However, this example must not be construed to limit the present invention in any meaning.

### Example: Prediction of nutritional requirements of Takifugu rubripes

In this example, proteins of *Takifugu rubripes* and *Homo sapience* were annotated with functions on the basis of amino acid sequences of the proteins predicted from the genome information of the organisms, metabolic pathways present in *Takifugu rubripes* and *Homo sapience* were identified and compared on the basis of the result of the annotation, and thereby candidates for nutrients specifically and strongly required by *Takifugu rubripes* were determined. The procedure and result are shown below.

The amino acid sequences data of all the proteins of *Homo sapiens* and *Takifugu rubripes* described in the FASTA format were obtained from the ftp site of Ensembl (http://asia.ensembl.org/info/data/ftp/index.html). The obtained amino acid sequences data were processed on the KEGG Automatic Annotation Server (KAAS) of iKeg (local server of KEGG) to collectively perform annotation of the proteins on the basis of KEGG Orthology (KO).

By using the free statistical analysis software "R" (http://www.r-project.org/), KO common to *Homo sapience* and *Takifugu rubripes* and KO specific to either one of them were distinguished.

The distinguished KO were mapped on metabolic pathways by using KEGG mapper (http://www.genome.jp/kegg/mapper.html), so that the metabolic pathways common to *Takifugu rubripes* and *Homo sapience* and metabolic pathways specific to either one of them could be recognized.

With taking the details of the metabolic pathways into consideration, candidates for nutrients strongly required by *Takifugu rubripes* were determined on the basis of the metabolic pathways specific to either one of *Takifugu rubripes* and *Homo sapience.* Specifically, when a metabolic pathway generating a metabolite important for growth or biological activities of *Homo sapience* was deficient in *Takifugu rubripes,* or when supply of a metabolite serving as a substrate of a metabolic pathway specific to *Takifugu rubripes* was insufficient, it was concluded that the metabolite and/or a metabolite generated from the metabolite by further metabolism are/is necessary to be acquired from the outside, and it was selected as candidate(s) for nutrient(s) strongly required by *Takifugu rubripes.* The candidates for nutrients strongly required by *Takifugu rubripes* are shown in Table 1.

As described above, nutritional requirements of fish was able to be determined on the basis of the amino acid sequences of proteins deduced from genome information.

[Table 1]

**Table 1**

| enzyme name | EC | human | torafugu | | nutrients | category |
|---|---|---|---|---|---|---|
| oligo-1,6-glucosidase | 3.2.1.10 | + | - | | 1,6 bond-hydrolysed carbon hydrate | sugar |
| gluconokinase | 2.7.1.12 | - | + | | D-gluconate, D-glucono-1,5-lactone | sugar |
| pepsin A | 3.4.23.1 | + | - | | peptide | amino acid |
| delta-6 desaturase | 1.14.19.- | + | - | | DHA, EPA, delta-6 unsaturated fatty acid | lipid |
| elongation of very long chain fatty acids protein 2 | 2.3.1.- | + | - | | DHA, EPA | lipid |
| pantetheine hydrolase | 3.5.1.92 | + | - | | pantothenate | vitamin |
| retinol dehydrogenase 11 | 1.1.1.- | + | - | | 11cis retinal | vitamin |

### Industrial Applicability

According to the present invention, the nutritional requirements of fish can be determined. Therefore, according to the present invention, formula feed that meets the nutritional requirements of fish can be provided.

## Claims

1. A method for determining nutritional requirements of fish, comprising:
(1) comparing the amino acid sequences of proteins predicted from genome information of fish with the amino acid sequences of known proteins thereby to estimate the functions of the respective proteins of the fish;
(2) identifying a metabolic pathway present in the fish on the basis of the estimated functions of the respective proteins; and
(3) determining nutritional requirements of the fish on the basis of a difference between the identified metabolic pathway present in the fish and a metabolic pathway present in a reference organism,
wherein the reference organism is a mammal or a bird.

2. The method according to claim 1, which comprises: comparing the amino acid sequences of proteins predicted from genome information of the reference organism with the amino acid sequences of known proteins thereby to estimate the functions of the respective proteins of the reference organism; and identifying the metabolic pathway present in the reference organism on the basis of the estimated functions of the respective proteins.

3. The method according to claim 1 or 2, wherein when a certain metabolic pathway is not present in the fish, but it is present in the reference organism, it is determined that the fish strongly requires a metabolite generated via the metabolic pathway.

4. The method according to any one of claims 1 to 3, wherein the amino acid sequences are described in the FASTA format.

5. The method according to any one of claims 1 to 4, wherein the functions of the respective proteins are estimated by homology search with known proteins using BLAST.

6. The method according to any one of claims 1 to 5, wherein the functions of the respective proteins are estimated by annotation based on KEGG Orthology (KO).

7. The method according to any one of claims 1 to 6, wherein the estimated functions of the respective proteins are mapped on a metabolic pathway.

8. The method according to any one of claims 1 to 7, wherein the reference organism is *Homo sapience.*

9. A method for producing feed for fish, which comprises: determining metabolite(s) strongly required by the fish according to the method according to any one of claims 3 to 8; and adding the metabolite(s) to a raw material of feed.
